# EUROPEAN PATENT SPECIFICATION

(11) **EP 2 745 857 B1**
(45) Date of publication and mention of the grant of the patent: **07.08.2019**
(21) Application number: 12824361.5
(22) Date of filing: 16.08.2012
(51) Int. Cl.: A61L 31/12, A61L 31/14

(54) **MULTILAYER DEGRADABLE STENT WITH SHAPE MEMORY PROPERTY AND PREPARATION METHOD THEREOF**
MEHRLAGIGER ABBAUBARER STENT MIT FORMGEDÄCHTNIS UND HERSTELLUNGSVERFAHREN DAFÜR
ENDOPROTHÈSE DÉGRADABLE MULTICOUCHE AVEC PROPRIÉTÉ DE MÉMOIRE DE FORME ET PROCÉDÉ DE PRÉPARATION DE CELUI-CI

(30) Priority: 17.08.2011 CN 201110236013
(43) Date of publication of application: 25.06.2014
(73) Proprietor: Shanghai MicroPort Medical (Group) Co., Ltd., Shanghai 201203 (CN)
(72) Inventor: CHEN, Baoai, Shanghai 201203 (CN); MENG, Juan, Shanghai 201203 (CN); SHI, Xiufeng, Shanghai 201203 (CN); LUO, Qiyi, Shanghai 201203 (CN)
(74) Representative: Dantz, Jan Henning
(86) International application number: PCT/CN2012/080228
(87) International publication number: WO 2013/023610

(56) References cited:
- CN-A- 1 386 478
- CN-A- 1 857 742
- CN-A- 101 219 069
- CN-A- 102 247 623
- KR-A- 20090 010 607
- US-A- 5 443 458
- US-A1- 2009 012 607
- US-A1- 2009 030 507
- US-B2- 7 731 740
- XUE L ET AL: "Biodegradable shape-memory block co-polymers for fast self-expandable stents", BIOMATERIALS, ELSEVIER SCIENCE PUBLISHERS BV., BARKING, GB, vol. 31, no. 32, 1 November 2010 (2010-11-01), pages 8132-8140, XP027259517, ISSN: 0142-9612 [retrieved on 2010-08-17]
- Small ET AL: "Biomedical applications of thermally activated shape memory polymers", Journal of Materials Chemistry, 1 January 2010 (2010-01-01), page 3356, XP055179485, DOI: 10.1039/b923717h Retrieved from the Internet: URL:https://e-reports-ext.llnl.gov/pdf/372 172.pdf
- VENKATRAMAN S S ET AL: "Biodegradable stents with elastic memory", BIOMATERIALS, ELSEVIER SCIENCE PUBLISHERS BV., BARKING, GB, vol. 27, no. 8, 1 March 2006 (2006-03-01), pages 1573-1578, XP027950913, ISSN: 0142-9612 [retrieved on 2006-03-01]
- MANI ET AL: "Coronary stents: A materials perspective", BIOMATERI, ELSEVIER SCIENCE PUBLISHERS BV., BARKING, GB, vol. 28, no. 9, 14 January 2007 (2007-01-14), pages 1689-1710, XP005830617, ISSN: 0142-9612, DOI: 10.1016/J.BIOMATERIALS.2006.11.042

## Description

### Technical Field

The present invention relates to the field of medical instrument, specifically, to a multilayer degradable stent with shape memory property and preparation method thereof.

### Background

When a blockage or stenosis occurs in a human blood vessel, stent needs to be implanted to expand this vessel. The support provided by the stent thus allows the blood to flow through the vessel normally again. The prevalent stents used clinically nowadays are permanent metal stents which however may interfere with surgical revascularization, prevent collateral circulation formation, and inhibit positive remodeling of the vessel. Therefore, novel provisional and degradable stents made of a degradable polymer material become hot research topic.

A degradable stent as one to be implanted into a human body must have a sufficiently high support strength and low elastic retraction, because a sufficiently high support strength is necessary to support the vessel and at the same time, it is desirable that the stent retracts as little as possible after being expanded in order to prevent deformation, displacement or fall-off of the stent once it is implanted.

Most of the present biodegradable stents are made of polymer materials and have poor support effect on blood vessels due to their low elastic modulus. Meanwhile, polymer materials tend to retract significantly after being expanded, making it easy for the implanted stent to move places or fall off.

US patent No. 7,731,740 (Polymer-based stent assembly) utilizes the shape memory property of polymer materials to reduce the retraction of an expanded stent, wherein the initial outer diameter of the degradable stent is designed to be the same as the operating outer diameter so that when the stent in a compressed and grasped state is implanted into a human body, it will still remember its initial size and after balloon inflation, it will be maintained at the operating outer diameter (i.e. the initial outer diameter) without producing significant retraction. However, this patent uses a single or copolymerized polymer material which requires a temperature higher than human body temperature to activate the shape memory property of the material. The effect brought by this temperature on human tissues make the biosafety of the stent a problem.

In order to reduce the retraction of a stent after being expanded, biodegradable polymer materials with shape memory properties are used to prepare stents in the prior art, but these materials often require a temperature higher than the body temperature to activate the shape memory property, bringing certain problems to the safety of the stent. A part of the polymer materials which can realize their shape memory properties at or below body temperature usually have strengths too low to meet the requirement on stent supporting forces. ENKATRAMAN S S ET AL: "Biodegradable stents with elastic memory",BIOMATERIALS, ELSEVIER SCIENCE PUBLISHERS BV., BARKING, GB, vol. 27, no. 8, 1 March 2006 (2006-03-01), pages 1573-1578 discloses bilayered fully biodegradable stents made from a layer of PLLA - which provides compression strength - and a layer of PLGA which has shape memory properties where PLGA has glass transition temperature higher than human body temperature.

In light of the above, there is a demand in this field for a novel degradable stent which not only meets the requirements of low retraction after expansion and resistance to deformation, but also has a shape memory property capable of being activated at body temperature and an excellent mechanical support property.

### Summary of the Invention

To solve the foresaid technical problem, the present invention provides a multilayer degradable stent with shape memory property prepared by combining a shape memory material with a material having higher strength. Especially, the present invention solves both of the technical problems, namely, retraction after the stent being expanded and insufficient support force, under the circumstances that no extra heat is introduced. It is contemplated in the present invention that the structure of the stent can be composed of two or more layers, wherein the material for one layer is a degradable polymer having shape memory property at body temperature which effectively reduces the retraction after the stent being expanded and requires no additional heat; and the material for another layer is a degradable metal or a degradable polymer having higher elastic modulus which can provide effective support for the vessel. The two layers of material can be either inner layer or outer layer with respect to one another. The stent according the present invention has small retraction after expansion and does not require introducing external heat. In addition, the stent according the present invention has a support force high enough to support narrowed blood vessels. After the degradation time is reached, the stent can rapidly degrade and be eliminated from the body through metabolic pathways, so the possibilities for a complication such as inflammation and restenosis to occur are effectively reduced.

In a multilayer degradable stent with shape memory property of the present invention, one layer is shape memory layer composed of a degradable material with shape memory property, and another layer is support layer composed of a degradable material with higher strength. The stent is compressed and grasped and then delivered to the lesion site before expanded by balloon to the operating diameter. Meanwhile, the shape memory property of the inner layer of the stent is activated at body temperature so that a propelling force is generated to maintain the stent at its initial shape (i.e., the operating shape in the blood vessel), effectively reducing the retraction of the stent after balloon inflation. The support layer of the stent can effectively support the vessel and prevent deformation and collapse. After the degradation time is reached, the stent body can rapidly degrade and be eliminated from the body through metabolic pathways. There is no introduction of external heat during the entire process, and the safety in stent usage is thus effectively guaranteed.

The technical solutions of the present invention are as follows.

A multilayer degradable stent with shape memory property, characterized in that it is composed of two or more layers of degradable material, wherein one layer is of a degradable polymer material with shape memory property, i.e., shape memory layer, and another layer is of a degradable metal material or a degradable polymer material with excellent radial support capability, i.e., support layer. The two layers of material can be either inner layer or outer layer with respect to one another.

The cut size of the stent is the size for use in a human body, and also a first shape of the stent. The stent is then compressed and grasped onto a balloon to be presented in a compressed and grasped state, and also a second shape of the stent. The stent is delivered into a human body in the compressed and grasped state, and is mechanically expanded by balloon inflation after it reaches the lesion site. Meanwhile, the shape memory property of the material in the shape memory layer of the stent is activated at human body temperature so that the stent would recall its memory of the first shape and be maintained in the first shape at body temperature, and in turn, be kept at the lesion site. As a result, the stent retraction after expansion is small.

The material of the shape memory layer is selected from degradable high polymers having a glass transition temperature (Tg) from 27°C to 37°C, including but not limited to a blend or copolymer of one or more of polylactic acid (PLA), polycaprolactone (PCL), polyglycolic acid (PGA), polyorthoesters, polyanhydrides and the like. The blending manner includes but is not limited to solution blending and melt blending. The copolymerization manner includes but is not limited to graft copolymerization, block copolymerization, random copolymerization and the like. The ratio by mass between components in a blend or copolymer varies from 1:1 to 1:20 so long as the modified material has the desired Tg. Since the shape memory activation temperatures of these materials are generally close to their glass transition temperatures, a polymer material having a Tg as specified above can be activated at body temperature so as to perform its shape memory property.

The biodegradable material of the support layer comprises a biodegradable metal material and a biodegradable polymer material, wherein the biodegradable metal material includes but is not limited to iron, magnesium or magnesium alloy; and the biodegradable polymer material includes but is not limited to poly-L-lactic acid (L-PLA), poly-D-lactic acid (D-PLA), polyglycolic acid (PGA), polytrimethylene carbonate (PTMC), poly-p-dioxanone (PPDO), polyamino acid derived carbonate, polyorthoester (POE) and the like, and a blend or copolymer formed therewith.

The preparation method of the multilayer degradable stent according to the present invention is realized by means comprising but not limited to twin-screw co-extrusion, surface spray coating, mold forming, compression forming, solvent adhesion and the like, wherein twin-screw co-extrusion means co-extruding materials from each layer of the multilayer stent so as to produce a tubular product with layered materials and strong adhesion between layers, and laser cutting the tubular product to provide the multilayer stent; surface spray coating means first preparing a tubular product of the material of the inner layer, then sequentially spraying onto the surface of the tubular product the material of the second layer, of the third layer and so on until arriving at a predetermined number of layers, and finally laser cutting the prepared tubular product to provide the multilayer stent; mold forming means different materials being formed in a layered mold to eventually provide the multilayer stent; compression forming means alternately arranging the layers of different materials to produce a sheet and rolling the sheet to form the stent; and solvent adhesion means preparing films of different degradable materials separately, forming a complete piece of film by adhering the films with solvent and rolling the film into filament which is convolved to form the multilayer stent.

The technical effects of the present invention are as follows.

In the multilayer degradable stent of the present invention, the material of the shape memory layer endows shape memory property to the stent at body temperature. After balloon inflation, the stent basically does not retract and is maintained at the predetermined operating outer diameter. The material of the support layer provides sufficient support strength for the stent, enabling the stent to effectively support the blood vessel walls after being implanted. After the multilayer stent is implanted, it has little retraction after expansion and high radial strength that can effectively support the vessel at the lesion site.

Due the degradability of the entire stent, after the degradation time is reached, the stent can rapidly degrade and be eliminated from the body through metabolic pathways, so the possibilities for a complication such as inflammation and restenosis to occur are effectively reduced.

In addition, because the shape memory property activation temperature of the material in the shape memory layer is body temperature, the entire process is completed at a human body temperature with no need to introduce any additional heat, and the biosafety of the stent is guaranteed.

### Detail Description of the Invention

To help further understand the present invention, the preferred embodiments of the present invention will be described below in conjunction with Examples. These descriptions are only used to exemplify the features and advantages of the present invention, but not to limit the scope of protection of the present invention.

### Example I

A multilayer degradable stent with shape memory property at body temperature is prepared by a method comprising:
PLCL8218 shape memory particles having a Tg of 30°C are prepared by copolymerizing poly-L-lactic acid (PLLA) and poly-ε-caprolactone (PCL) at a ratio of 82:18, and are used as the material of the inner layer of the stent. PLGA9010 particles having higher elastic modulus and support strength and prepared by copolymerizing poly (lactic acid-glycolic acid) at a ratio of 90:10 are used in the support layer as the outer layer of the stent. PLCL8218 and PLGA9010 particles are co-extruded with a twin-screw extruder to produce a degradable tubular product having an outer diameter of 3.0mm and a wall thickness of 0.15mm. A two-layer stent with an inner layer of PLCL material and an outer layer of PLGA material is obtained by laser cutting the tubular product.

The stent is compressed and grasped onto a suitable balloon, and then delivered to the vascular lesion site with an original inner diameter of 2.7mm. After balloon inflation, it is observed that the narrowed blood vessel is propped open in 10s. At the same time, the shape memory property of the inner layer material is activated at body temperature (37°C). The stent recalls its memory of the cutting shape having an outer diameter of 3.0mm and is maintained in this shape. After the balloon is withdrawn upon completion of the stent expansion, no apparent retraction or displacement of the stent is observed. During the entire process of test, no deformation, rupture or fall-off of the stent occurs and the blood vessel is effectively supported. Four months after implantation, endothelialisation of the stent starts; in six months, endothelialisation is almost finished; and 12 months later, the stent is almost completely degraded. There is no inflammation or restenosis observed in the entire process from implantation to complete degradation of the stent.

### Example II

Shape memory particles produced by melt blending poly-DL-lactic acid (PDLA) particles and polydioxanone (PPDO) particles and granulating the blends are utilized as the outer layer material of the stent in this example. PDLA and PPDO are at a mass ratio w₁:w₂=52:48, and the obtained material with shape memory property has Tg=30°C. The shape memory particles for the outer layer are dissolved in a co-solvent for PDLA and PPDO, hexafluoroisopropanol (HFIP) to give a solution.

Degradable metal material, magnesium alloy is used as the material of the support layer, also the inner layer of the stent. Magnesium alloy is extruded to produce a tubular product having an outer diameter of 2.9mm and a wall thickness of 0.1 mm, on the surface of which the solution of the outer layer material is sprayed. After spray coating, the tubular product is left in a vacuum chamber to allow evaporation of the solvent and dry. After complete drying, the spray coating and drying cycle can be repeated until a thickness of 0.1mm of the dried coating layer is reached. The final tubular product has an outer diameter of 3.0mm and a wall thickness of 0.2mm. A two-layer stent with an inner layer of magnesium alloy and an outer layer of PDLA-PPDO is obtained by laser cutting the prepared tubular product.

The support strength of thus-obtained two-layer stent is over 100KPa and the elastic retraction rate after expansion of the same is below 5%. The stent is compressed and grasped onto a suitable balloon, and then delivered to the vascular stenosis site with an original inner diameter of 2.6mm. After arriving at the lesion site and upon balloon inflation, it is observed that the narrowed blood vessel is propped open in 10s. At the same time, the shape memory property of the outer layer material is activated at body temperature (37°C). The stent recalls its memory of the cutting shape having an outer diameter of 3.0mm and is maintained in this shape. After the balloon is withdrawn, there is nearly none retraction of the stent. During the entire process, no deformation, displacement or rupture of the stent occurs either. The stent can effectively provide support for the vascular lesion site for about 4 months, and 12 months later, the degradation of the stent is almost completed. No complication such as inflammation, restenosis or the like is observed

### Example III

This example is basically the same as Example I, except that: the multilayer stent is formed by compressing PLCL8218 material and PLGA9010 material into sheets separately, and folding and welding the produced sheets to form a cylindrical product, wherein PLCL8218 is used for the inner layer and PLGA9010 for the outer layer. The multilayer stent is obtained by cutting the cylindrical product.

After being compressed and grasped, the stent is delivered into a human body and effectively props the narrowed blood vessel open through balloon inflation. After the balloon is withdrawn, the stent has little retraction due to the shape memory property of the inner layer material. The stent provides effective support for the vessel within 6 months, and 12 months later, the degradation of the stent is almost completed and the vascular lesion site is healed.

## Claims

1. A multilayer degradable stent with shape memory property, **characterized in that** it is composed of two or more layers of degradable material, wherein one layer is of a degradable polymer material with shape memory property, i.e., shape memory layer, and another layer is of a degradable metal material or a degradable polymer material with excellent radial support capability, i.e., support layer,
wherein the material of the shape memory layer is a degradable polymer having a glass transition temperature (Tg) from 27°C to 37°C.

2. The multilayer degradable stent according to claim 1, wherein the shape memory layer and the support layer are either inner layer or outer layer with respect to one another.

3. The multilayer degradable stent according to claim 1, wherein the degradable polymer of the shape memory layer is selected from a blend or copolymer of one or more of polylactic acid (PLA), polycaprolactone (PCL), polyglycolic acid (PGA), polyorthoesters and polyanhydrides.

4. The multilayer degradable stent according to claim 3, wherein the blend is obtained by solution blending or melt blending.

5. The multilayer degradable stent according to claim 3, wherein the copolymer is obtained by graft copolymerization, block copolymerization, or random copolymerization.

6. The multilayer degradable stent according to any one of claims 3-5, wherein the ratio by mass between components in the blend or copolymer is from 1: 1 to 1:20 so that the modified material has the desired Tg.

7. The multilayer degradable stent according to claim 1, wherein the biodegradable metal material is selected from iron, magnesium or magnesium alloy.

8. The multilayer degradable stent according to claim 1, wherein biodegradable polymer material of the support layer is selected from poly-L-lactic acid (L-PLA), poly-D-lactic acid (D-PLA), polyglycolic acid (PGA), polytrimethylene carbonate (PTMC), poly-p-dioxanone (PPDO), polyamino acid derived carbonate, polyorthoester (POE), and a blend or copolymer formed therewith.

9. A method for preparing the multilayer degradable stent according to any one of the preceding claims, said method being realized by a means selected from twin-screw co-extrusion, surface spray coating, mold forming, compression forming, and solvent adhesion, wherein twin-screw co-extrusion means co-extruding materials from each layer of the multilayer stent so as to produce a tubular product with layered materials and strong adhesion between layers, and laser cutting the tubular product to provide the multilayer stent; surface spray coating means first preparing a tubular product from the material of the inner layer, then sequentially spraying onto the surface of the tubular product the material of the second layer, of the third layer and so on until arriving at a predetermined number of layers, and finally laser cutting the prepared tubular product to provide the multilayer stent; mold forming means different materials being formed in a layered mold to eventually provide the multilayer stent; compression forming means alternately arranging the layers of different materials to produce a sheet and rolling the sheet to form the stent; and solvent adhesion means preparing films from different degradable materials separately, forming a complete piece of film by adhering the films with solvent and rolling the film into filament which is convolved to form the multilayer stent.

## Patentansprüche

1. Mehrschichtiger abbaubarer Stent mit Formgedächtniseigenschaft, **dadurch gekennzeichnet, dass** er aus zwei oder mehr Schichten aus abbaubarem Material zusammengesetzt ist, wobei eine Schicht aus einem abbaubaren Polymermaterial mit Formgedächtniseigenschaft, d.h. einer Formgedächtnisschicht, besteht und die andere Schicht aus einem abbaubaren Metallmaterial oder einem abbaubaren Polymermaterial mit ausgezeichneter radialer Stützfähigkeit, d.h. einer Stützschicht, besteht,
wobei das Material der Formgedächtnisschicht ein abbaubares Polymer ist, das eine Glasübergangstemperatur (Tg) von 27 °C bis 37 °C aufweist.

2. Mehrschichtiger abbaubarer Stent nach Anspruch 1, wobei die Formgedächtnisschicht und die Stützschicht in Bezug zueinander entweder eine innere Schicht oder eine äußere Schicht sind.

3. Mehrschichtiger abbaubarer Stent nach Anspruch 1, wobei das abbaubare Polymer der Formgedächtnisschicht aus einem Gemisch oder Copolymer von einem oder mehrerem aus Polymilchsäure (PLA), Polycaprolacton (PCL), Polyglycolsäure (PGA), Polyorthoestern und Polyanhydriden ausgewählt wird.

4. Mehrschichtiger abbaubarer Stent nach Anspruch 3, wobei das Gemisch durch Lösungsmischen oder Schmelzmischen erhalten wird.

5. Mehrschichtiger abbaubarer Stent nach Anspruch 3, wobei das Copolymer durch Pfropfcopolymerisation, Blockcopolymerisation oder statistische Copolymerization erhalten wird.

6. Mehrschichtiger abbaubarer Stent nach einem der Ansprüche 3 bis 5, wobei das Massenverhältnis zwischen den Komponenten in dem Gemisch oder Copolymer von 1:1 bis 1:20 beträgt, sodass das modifizierte Material die gewünschte Tg aufweist.

7. Mehrschichtiger abbaubarer Stent nach Anspruch 1, wobei das biologisch abbaubare Metallmaterial aus Eisen, Magnesium oder Magnesiumlegierung ausgewählt wird.

8. Mehrschichtiger abbaubarer Stent nach Anspruch 1, wobei das biologisch abbaubare Polymermaterial der Stützschicht aus Poly-L-Milchsäure (L-PLA), Poly-D-Milchsäure (D-PLA), Polyglycolsäure (PGA), Polytrimethylencarbonat (PTMC), Poly-p-Dioxanon (PPDO), einem von Polyaminosäuren abgeleiteten Carbonat, Polyorthoester (POE) und einem damit gebildeten Gemisch oder Copolymer ausgewählt wird.

9. Verfahren zur Herstellung des mehrschichtigen abbaubaren Stents nach einem der vorhergehenden Ansprüche, wobei das Verfahren durch ein Mittel verwirklicht wird, das aus Doppelschnecken-Co-Extrusion, Oberflächensprühbeschichtung, Formen in der Form, Kompressionsformen und Lösungsmittelverklebung ausgewählt wird, wobei mit Doppelschnecken-Co-Extrusion das Co-Extrudieren von Materialien jeder Schicht des mehrschichtigen Stents gemeint ist, sodass ein rohrförmiges Produkt mit geschichteten Materialien und einer starken Haftung zwischen den Schichten erzeugt wird, wobei das rohrförmige Produkt lasergeschnitten wird, um den mehrschichtigen Stent bereitzustellen; wobei mit Oberflächensprühbeschichtung gemeint ist, dass zuerst ein rohrförmiges Produkt aus dem Material der inneren Schicht hergestellt wird, anschließend nacheinander das Material der zweiten Schicht, der dritten Schicht und so weiter auf die Oberfläche des rohrförmigen Produkts gesprüht wird, bis eine vorbestimmte Anzahl an Schichten erreicht ist, und schließlich das hergestellte rohrförmige Produkt lasergeschnitten wird, um den mehrschichtigen Stent bereitzustellen; wobei mit Formen in der Form gemeint ist, dass verschiedene Materialien in einer geschichteten Form geformt werden, um letztlich den mehrschichtigen Stent bereitzustellen; wobei mit Kompressionsformen gemeint ist, dass die Schichten aus verschiedenen Materialien abwechselnd angeordnet werden, um eine Platte zu bilden, und dass die Platte gewalzt wird, um den Stent zu bilden; und wobei mit Lösungsmittelverklebung gemeint ist, dass Filme aus verschiedenen abbaubaren Materialien separat hergestellt werden, ein komplettes Stück Film hergestellt wird, indem die Filme mit einem Lösemittel miteinander verklebt werden und indem der Film zu einem Filament gewalzt wird, das zusammengerollt wird, um den mehrschichtigen Stent zu bilden.

## Revendications

1. Endoprothèse dégradable multicouche avec propriété de mémoire de forme, **caractérisée en ce qu'**elle est composée de deux couches ou plus de matériau dégradable, dans laquelle une couche est en matériau polymère dégradable doté d'une propriété de mémoire de forme, c'est-à-dire, une couche à mémoire de forme, et une autre couche est en matériau en métal dégradable ou matériau polymère dégradable doté d'une excellente capacité de soutien radial, c'est-à-dire une couche de soutien,
dans laquelle le matériau de la couche à mémoire de forme est un polymère dégradable ayant une température de transition vitreuse (Tg) de 27 °C à 37 °C.

2. Endoprothèse dégradable multicouche selon la revendication 1, dans laquelle la couche à mémoire de forme et la couche de soutien sont soit une couche intérieure soit une couche extérieure l'une par rapport à l'autre.

3. Endoprothèse dégradable multicouche selon la revendication 1, dans laquelle le polymère dégradable de la couche à mémoire de forme est choisi parmi un mélange ou copolymère d'un ou de plusieurs du polyacide lactique (PLA), de la polycaprolactone (PCL), du polyacide glycolique (PGA), des polyorthoesters et des polyanhydrides.

4. Endoprothèse dégradable multicouche selon la revendication 3, dans laquelle le mélange est obtenu par mélange de solution ou mélange en fusion.

5. Endoprothèse dégradable multicouche selon la revendication 3, dans laquelle le copolymère est obtenu par une copolymérisation avec greffage, une copolymérisation séquencée, ou une copolymérisation statistique.

6. Endoprothèse dégradable multicouche selon l'une quelconque des revendications 3 à 5, dans laquelle le rapport en masse entre des composants du mélange ou copolymère varie de 1:1 à 1:20 de sorte que le matériau modifié ait la Tg souhaitée.

7. Endoprothèse dégradable multicouche selon la revendication 1, dans laquelle le matériau en métal biodégradable est choisi parmi le fer, le magnésium ou un alliage de magnésium.

8. Endoprothèse dégradable multicouche selon la revendication 1, dans laquelle le matériau polymère biodégradable de la couche de soutien est choisi parmi le polyacide L-lactique (L-PLA), le polyacide D-lactique (D-PLA), le polyacide glycolique (PGA), le polycarbonate de triméthylène (PTMC), la poly-p-dioxanone (PPDO), un carbonate dérivé de polyacides aminés, le polyorthoester (POE), et un mélange ou copolymère formé avec ceux-ci.

9. Procédé de préparation de l'endoprothèse dégradable multicouche selon l'une quelconque des revendications précédentes, ledit procédé étant réalisé par un moyen choisi parmi la co-extrusion double vis, le revêtement par pulvérisation de surface, la formation en moule, la formation par compression, et l'adhérence par solvant, dans lequel la co-extrusion double vis signifie la co-extrusion de matériaux à partir de chaque couche de l'endoprothèse multicouche de façon à produire un produit tubulaire avec des matériaux en couche et une adhérence forte entre les couches, et la découpe laser du produit tubulaire pour fournir l'endoprothèse multicouche ; le revêtement par pulvérisation de surface signifie tout d'abord la préparation d'un produit tubulaire à partir du matériau de la couche intérieure, puis la pulvérisation séquentielle sur la surface du produit tubulaire du matériau de la deuxième couche, de la troisième couche et ainsi de suite jusqu'à arriver à un nombre prédéterminé de couches, et enfin la découpe laser du produit tubulaire préparé pour fournir l'endoprothèse multicouche ; la formation en moule signifie que des matériaux différents sont formés dans un moule en couches pour finalement fournir l'endoprothèse multicouche ; la formation par compression signifie l'agencement en alternance des couches de matériaux différents pour produire une feuille et le laminage de la feuille pour former l'endoprothèse ; et l'adhérence par solvant signifie la préparation de films à partir de matériaux dégradables différents séparément, la formation d'un morceau complet de film en faisant adhérer les films avec un solvant et le laminage du film en un filament qui est convolué pour former l'endoprothèse multicouche.
